(19) Europäisches Patentamt
European Patent Office
Office européen des brevets

(11) EP 2 489 366 B1

# (12) EUROPEAN PATENT SPECIFICATION

(45) Date of publication and mention of the grant of the patent:
**17.02.2016 Bulletin 2016/07**

(21) Application number: **09850335.2**

(22) Date of filing: **12.10.2009**

(51) Int Cl.:
*A61K 38/55* (2006.01) *A61K 48/00* (2006.01)
*A61P 35/00* (2006.01) *A61K 38/00* (2006.01)
*A61K 38/57* (2006.01)

(86) International application number:
**PCT/CN2009/074410**

(87) International publication number:
**WO 2011/044721 (21.04.2011 Gazette 2011/16)**

## (54) USE OF SPINK6 GENE AND PROTEIN ENCODED THEREIN IN THE MANUFACTURE OF ANTI-TUMOR MEDICAMENTS

VERWENDUNG DES SPINK6-GENS UND DARIN CODIERTES PROTEIN ZUR HERSTELLUNG VON MEDIKAMENTEN GEGEN TUMOR

UTILISATION DU GÈNE SPINK6 ET DE LA PROTÉINE CODÉE PAR CELUI-CI DANS LA FABRICATION DE MÉDICAMENTS ANTI-TUMORAUX

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**22.08.2012 Bulletin 2012/34**

(73) Proprietor: **Fudan University**
**Shanghai 200433 (CN)**

(72) Inventors:
- **HUANG, Qingshan**
  **Shanghai 200433 (CN)**
- **WANG, Wei**
  **Shanghai 200433 (CN)**
- **HUANG, Jinjiang**
  **Shanghai 200433 (CN)**
- **LU, Hairong**
  **Shanghai 200433 (CN)**
- **LI, Guodong**
  **Shanghai 200433 (CN)**

(74) Representative: **Maikowski & Ninnemann**
**Postfach 15 09 20**
**10671 Berlin (DE)**

(56) References cited:
**WO-A1-99/55865** **WO-A1-2008/052238**
**WO-A1-2008/098720**

- **SONG S Y ET AL: "Expression of reversion-inducing-cysteine-rich protein with Kazal motifs (RECK) as a prognostic indicator in gastric cancer", EUROPEAN JOURNAL OF CANCER, PERGAMON PRESS, OXFORD, GB, vol. 42, no. 1, 1 January 2006 (2006-01-01), pages 101-108, XP027963741, ISSN: 0959-8049 [retrieved on 2006-01-01]**
- **KAZUMASA TAKENAKA ET AL: "Prognostic Significance of Reversion-Inducing Cysteine-Rich Protein With Kazal Motifs Expression in Resected Pathologic Stage IIIA N2 Non-Small-Cell Lung Cancer", ANNALS OF SURGICAL ONCOLOGY, SPRINGER-VERLAG, NE, vol. 12, no. 10, 1 October 2005 (2005-10-01), pages 817-824, XP019369584, ISSN: 1534-4681**
- **MARTIN C WAPENAAR ET AL: "The SPINK gene family and celiac disease susceptibility", IMMUNOGENETICS, SPRINGER, BERLIN, DE, vol. 59, no. 5, 27 February 2007 (2007-02-27), pages 349-357, XP019513572, ISSN: 1432-1211, DOI: 10.1007/S00251-007-0199-5**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 489 366 B1

Sequence Listing

<110>Fudan University

<120>Use of SPINK6 gene and the encoded protein for the preparation of an anti-tumor medicaments

<130>

<160>7

<170>PatentIn Version3.1

<210>1
<211>174
<212>DNA
<213>Homo sapiens

<400>1

cagggaggac aggttgactg tggtgagttc caggacacca aggtctactg cactcgggaa 60
tctaacccac actgtggctc tgatggccag acatatggca ataaatgtgc cttctgtaag 120
gccatagtga aaagtggtgg aaagattagc ctaaagcatc ctggaaaatg ctga 174

<210>2
<211>57
<212>PRT
<213>Homo sapiens

<400>2

```
Gln Gly Gly Gln Val Asp Cys Gly Glu Phe Gln Asp Thr Lys Val
                5                   10                  15
Tyr CYs Thr Arg Glu Ser Asn Pro His Cys Gly Ser Asp Gly Gln
                20                  25                  30
Thr Tyr Gly Asn Lys Cys Ala Phe Cys Lys Ala Ile Val Lys Ser
                35                  40                  45
Gly Gly Lys Ile Ser Leu Lys His Pro Gly Lys Cys
                50                  55
```

<210>3
<211>243
<212>DNA
<213>Homo sapiens

<400>3

atgaaactgt caggcatgtt tctgctcctc tctctggctc ttttctgctt tttaacaggt 60
gtcttcagtc agggaggaca ggttgactgt ggtgagttcc aggacaccaa ggtctactgc 120
actcgggaat ctaacccaca ctgtggctct gatggccaga catatggcaa taaatgtgcc 180
ttctgtaagg ccatagtgaa aagtggtgga aagattagcc taaagcatcc tggaaaatgc 240
tga 243

<210>4
<211>80
<212>PRT
<213>Homo sapiens

<400>4

Met Lys Leu Ser Gly Met Phe Leu Leu Leu Ser Leu Ala Leu Phe
5 10 15
Cys Phe Leu Thr Gly Val Phe Ser Gln Gly Gly Gln Val Asp Cys
20 25 30
Gly Glu Phe Gln Asp Thr Lys Val Tyr CYs Thr Arg Glu Ser Asn
35 40 45
Pro His Cys Gly Ser Asp Gly Gln Thr Tyr Gly Asn Lys Cys Ala
50 55 60
Phe Cys Lys Ala Ile Val Lys Ser Gly Gly Lys Ile Ser Leu Lys
65 70 75
His Pro Gly Lys Cys
80

<210>5
<211>254
<212>DNA
<213>Artificial sequence

<220>
<221>sp6

<400>5

gccatggcta aactgtcagg catgtttctg ctcctctctc tggctctttt ctgcttttta 60

acaggtgtct tcagtcaggg aggacaggtt gactgtggtg agttccagga caccaaggtc 120
tactgcactc gggaatctaa cccacactgt ggctctgatg gccagacata tggcaataaa 180
tgtgccttct gtaaggccat agtgaaaagt ggtggaaaga ttagcctaaa gcatcctgga 240
aaatgctgaa gctt 254

<210>6
<211>195
<212>DNA
<213>Artificial sequence

<220>
<221>spink6a

<400>6

ctcgagaaaa gacagggtgg acaggttgac tgtggtgagt tccaggaccc aaaggtctac 60
tgcactagag agtctaaccc acactgtggt tctgatggac agacttacgg caacaaatgt 120
gccttctgta aggccatagt taagtctggt ggaaagattt ctctgaagca tcctggaaag 180
tgctaatgag aattc 195

<210>7
<211>264
<212>DNA
<213>Artificial sequence

<220>
<221>spink6b

<400>7

ctcgagaaaa gaatgaaact gtcaggcatg tttctgctcc tctctctggc tcttttctgc 60
tttttaacag gtgtcttcag tcagggtgga caggttgact gtggtgagtt ccaggaccca 120
aaggtctact gcactagaga gtctaaccca cactgtggtt ctgatggaca gacttacggc 180
aacaaatgtg ccttctgtaa ggccatagtt aagtctggtg gaaagatttc tctgaagcat 240
cctggaaagt gctaatgaga attc 264

SEQUENCE LISTING

<110> Fudan University

<120> USE OF SPINK6 GENE AND PROTEIN ENCODED THEREIN IN THE MANUFACTURE OF ANTI-TUMOR MEDICAMENTS

<130> CHI102EP

<140> PCT/CN2009074410
<141> 2009-10-12

<160> 15

<170> PatentIn version 3.5

<210> 1
<211> 174
<212> DNA
<213> Homo sapiens

<400> 1

cagggaggac aggttgactg tggtgagttc caggacacca aggtctactg cactcgggaa 60
tctaacccac actgtggctc tgatggccag acatatggca ataaatgtgc cttctgtaag 120
gccatagtga aaagtggtgg aaagattagc ctaaagcatc ctggaaaatg ctga 174

<210> 2
<211> 57
<212> PRT
<213> Homo sapiens

<400> 2

Gln Gly Gly Gln Val Asp Cys Gly Glu Phe Gln Asp Thr Lys Val Tyr
1               5                   10                  15

Cys Thr Arg Glu Ser Asn Pro His Cys Gly Ser Asp Gly Gln Thr Tyr
            20                  25                  30

Gly Asn Lys Cys Ala Phe Cys Lys Ala Ile Val Lys Ser Gly Gly Lys
        35                  40                  45

Ile Ser Leu Lys His Pro Gly Lys Cys
    50                  55

<210> 3
<211> 243
<212> DNA
<213> Homo sapiens

<400> 3

atgaaactgt caggcatgtt tctgctcctc tctctggctc ttttctgctt tttaacaggt 60

gtcttcagtc agggaggaca ggttgactgt ggtgagttcc aggacaccaa ggtctactgc 120

actcgggaat ctaacccaca ctgtggctct gatggccaga catatggcaa taaatgtgcc 180

ttctgtaagg ccatagtgaa aagtggtgga aagattagcc taaagcatcc tggaaaatgc 240

tga 243

<210> 4
<211> 80
<212> PRT
<213> Homo sapiens

<400> 4

Met Lys Leu Ser Gly Met Phe Leu Leu Leu Ser Leu Ala Leu Phe Cys
1               5                   10                  15

Phe Leu Thr Gly Val Phe Ser Gln Gly Gly Gln Val Asp Cys Gly Glu
            20                  25                  30

Phe Gln Asp Thr Lys Val Tyr Cys Thr Arg Glu Ser Asn Pro His Cys
        35                  40                  45

Gly Ser Asp Gly Gln Thr Tyr Gly Asn Lys Cys Ala Phe Cys Lys Ala
    50                  55                  60

Ile Val Lys Ser Gly Gly Lys Ile Ser Leu Lys His Pro Gly Lys Cys
65                  70                  75                  80

<210> 5
<211> 254
<212> DNA
<213> Artificial sequence

<220>
<223> Synthetic gene fragment

<400> 5

```
gccatggcta aactgtcagg catgtttctg ctcctctctc tggctctttt ctgcttttta    60
acaggtgtct tcagtcaggg aggacaggtt gactgtggtg agttccagga caccaaggtc   120
tactgcactc gggaatctaa cccacactgt ggctctgatg gccagacata tggcaataaa   180
tgtgccttct gtaaggccat agtgaaaagt ggtggaaaga ttagcctaaa gcatcctgga   240
aaatgctgaa gctt                                                     254
```

<210> 6
<211> 195
<212> DNA
<213> Artificial sequence

<220>
<223> Synthetic gene fragment

<400> 6

```
ctcgagaaaa gacagggtgg acaggttgac tgtggtgagt tccaggaccc aaaggtctac    60
tgcactagag agtctaaccc acactgtggt tctgatggac agacttacgg caacaaatgt   120
gccttctgta aggccatagt taagtctggt ggaaagattt ctctgaagca tcctggaaag   180
tgctaatgag aattc                                                    195
```

<210> 7
<211> 264
<212> DNA
<213> Artificial sequence

<220>
<223> Synthetic gene fragment

<400> 7

```
ctcgagaaaa gaatgaaact gtcaggcatg tttctgctcc tctctctggc tcttttctgc    60
tttttaacag gtgtcttcag tcagggtgga caggttgact gtggtgagtt ccaggaccca   120
aaggtctact gcactagaga gtctaaccca cactgtggtt ctgatggaca gacttacggc   180
aacaaatgtg ccttctgtaa ggccatagtt aagtctggtg gaaagatttc tctgaagcat   240
cctggaaagt gctaatgaga attc                                          264
```

<210> 8
<211> 29
<212> DNA
<213> Artificial sequence

<220>
<223> Synthetic oligonucleotide

<400> 8
cggaattccg aatgaaactg tcaggcatg 29

<210> 9
<211> 26
<212> DNA
<213> Artificial sequence

<220>
<223> Synthetic oligonucleotide

<400> 9
ccaagctttc agcattttcc aggatg 26

<210> 10
<211> 26
<212> DNA
<213> Artificial sequence

<220>
<223> Synthetic oligonucleotide

<400> 10
cggaattcca gggaggacag gttgac 26

<210> 11
<211> 26
<212> DNA
<213> Artificial sequence

<220>
<223> Synthetic oligonucleotide

<400> 11
ccaagctttc agcattttcc aggatg 26

<210> 12
<211> 26
<212> DNA
<213> Artificial sequence

<220>
<223> Synthetic oligonucleotide

<400> 12
ccggtaccat gaaactgtca ggcatg 26

<210> 13
<211> 26
<212> DNA
<213> Artificial sequence

<220>
<223> Synthetic oligonucleotide

<400> 13
cgctcgagtc agcattttcc aggatg 26

<210> 14
<211> 26
<212> DNA
<213> Artificial sequence

<220>
<223> Synthetic oligonucleotide

<400> 14
ccggtaccca gggaggacag gttgac 26

<210> 15
<211> 26
<212> DNA
<213> Artificial sequence

<220>
<223> Synthetic oligonucleotide

<400> 15
cgctcgagtc agcattttcc aggatg 26

## Description

### FIELD OF INVENTION

The present invention relates to new use of *spink6* gene or its encoded protein. Specifically, the present invention relates to the use of the *spink6* gene or the encoded protein in the preparation of anti-liver cancer drugs.

### BACKGROUND OF INVENTION

The sequence of *spink6* gene is a human-derived gene sequence published by NCBI in 2006, which has been reported in detail in Martin C. Wapenaar, Alienke J. Monsuur et al, Immunogenetics, 2007, 59:349-357. Its encoded SPINK6 protein contains a Kazal domain and classified into the SERPIN family of serine protease inhibitors of the Kazal type.

To date, there is no other report on specific functions of the *spink6* gene and its encoded protein. Whether their applications in pharmaceutical field can be further studied is our main concern.

Cancer is currently one of the major diseases in humans, more and more investigators have turned their attentions to study tumor related genes, in order to fundamentally resolve the problem of the occurrence of tumor diseases in terms of genes. Whether the *spink6* gene and the encoded protein can be used to prepare a new drug for treating liver cancer will be very meaningful and clinically important.

### SUMMARY OF THE INVENTION

The technical problem to be resolved in the present disclosure is a use of a gene or its encoded protein thereof in the manufacture of an anti-liver cancer drug.

The technical concept of the disclosure is as follows:

> The SPINK6 protein belongs to a serine protease inhibitor, containing a signal peptide of 23 amino acids. The SPINK6 protein could be secreted extracellularly and acted via the receptors on the cell membrane. Therefore, we speculated that the SPINK6 protein plays a role via the transmembrane serine protease on the cell membrane. The known serine proteases are associated with many kinds of tumors and cancers. For example, Matriptase is a type II transmembrane serine protease and is significantly high expressed in epithelial ovarian cancer (Michael D. Oberst, Michael D. et al. Clinical Cancer Research, 2002 April, Vol. 8, 1101-1107); Hepsin is a transmembrane serine protease, and is significantly high expressed in prostate cancer patients (Klezovitch J., Chevillet J. et al. Cancer Cell, 2004, Volume 6, Issue 2, Pages 185-195). Therefore, it is speculated that *spink6* gene sequence and the encoded protein thereof may also have tumor suppressing activity.

To this end, through extensive researches, we confirmed that *spink6* gene, and the encoded proteins with or without the signal peptide of 23 amino acids can inhibit the growth and malignant of tumor cells, affect strongly the cell cycle. Therefore, *spink6* gene and the encoded SPINK6 protein with or without the signal peptide of 23 amino acids have the application prospects in the preparation of anti-liver cancer drugs.

The gene described herein has the nucleotide sequence designated as SED ID NO:1 or SED ID NO:3.

The protein encoded by the gene described herein has the amino acid sequence designated as SED ID NO:2 or SED ID NO:4.

The use of the protein described herein in the manufacture of an anti-liver cancer drug is in the form of a pharmaceutical composition.

The term "SPINK6 protein" as used herein refers to the protein having the amino acid sequence of human *spink6* designated as SED ID NO:2 or SED ID NO:4.

The difference between the nucleotide sequence designated as SED ID NO:1 and the nucleotide sequence designated as SED ID NO:3 lies in that the nucleotide sequence designated as SED ID NO:3 further includes a sequence of 69 nucleotides (i.e., nucleotide positions 1-69 of SED ID NO:3) encoding a signal peptide of 23 amino acids at 5' end of the nucleotide sequence designated as SED ID NO:1.

The difference between the amino acid sequence designated as SED ID NO:2 and the amino acid sequence designated as SED ID NO:4 lies in that the amino acid sequence designated as SED ID NO:4 further includes a signal peptide of 23 amino acids (i.e., amino acid positions 1-23 of SED ID NO:4) at N-terminus of the amino acid sequence designated as SED ID NO:2.

The disclosure also relates to a vector comprising a *spink6* polynucleotide according to the present invention, and a host cell produced via gene engineering by the vector or the sequence encoding SPINK6 protein- according to the present disclosure as well as a method for producing the polypeptide described herein by recombinant technology.

The polynucleotide sequence according to the present disclosure can be used to express or produce the recombinant SPINK6 protein through conventional recombinant DNA technology, such as the technology reported in the literature (Science, 1984; 224: 1431). In general, the following steps are included:

(1) transforming or transducing a suitable host cell including E. coli, yeast or CHO with the polynucleotide encoding SPINK6 protein or a recombinant expression vector containing the polynucleotide according to the present disclosure
(2) culturing the host cell in a suitable medium, which may be routinely chosen according to Molecular Cloning: A Laboratory Manual (3), Joe Sambrook, et al.;
(3) isolating and purifying the protein from the medium or the cell.

The recombinant SPINK6 protein has various applications. These applications include but are not limited to directly as a drug for the treatment of the diseases caused by dysfunction or loss of *spink6,* such as a variety of malignant tumors and abnormal cell proliferation, etc.

The protein according to the present disclosure can be used in combination with an appropriate pharmacologically acceptable carrier.

The carrier includes but is not limited to: a diluent, an excipient such as water, etc.; a filler such as starch, sucrose, etc.; an adhesive such as a fiber derivative, alginate, gelatin and polyvinylpyrrolidone; a wetting agent such as glycerol; a disintegrating agent such as agar, calcium carbonate and calcium bicarbonate; an absorption enhancer such as a quaternary ammonium compound; a surfactant such as cetyl alcohol; an adsorption carrier such as kaolin and bentonite; a lubricant such as talc, calcium and magnesium stearate, polyethylene glycol, and other.

The pharmaceutical composition can be administered to a patient in need of treatment in a convenient manner, such as via local, intravenous, intraperitoneal, intramuscular, subcutaneous, intranasal or intradermal administration route. The dosage of SPINK6 protein administered to the patients is generally 0.5 ~ 2mg/kg body weight/day, and the specific range will depend on many factors, such as the administration manner, the health condition of the patient to be treated and the judgment of physician.

In addition, the polynucleotide of *spink6* can also be used for various therapeutic purposes. Gene therapy techniques can be used to treat abnormalities of cell proliferation, development or metabolism caused by no expression of SPINK6 protein or the expression of abnormal/inactive SPINK6 protein. A recombinant gene therapy vector (such as a viral vector) can be designed to express SPINK6 protein, in order to compensate abnormalities of cell proliferation, development or metabolism caused by insufficient expression of intrinsic SPINK6 protein or the expression of abnormal/inactive SPINK6 protein. Therefore, the recombinant gene therapy vector can be used to treat the diseases caused by abnormalities of SPINK6 expression or activity, such as liver cancer. The expression vector derived from a virus, such as retrovirus, adenovirus, adeno-associated virus, herpes simplex virus, parvovirus and the like, can be used to transfer *spink6* gene into the cell. The method for constructing the recombinant virus vector carrying the *spink6* gene can be found in the literatures (Sambrook, et al.). Furthermore, the recombinant *spink6* gene can be packaged into a liposome and transferred into the cell.

The method for introducing the polynucleotide into a tissue or cell includes direct polynucleotide injection into the body tissue; or at first introducing the polynucleotide into the cell *in vitro* via a vector (such as a virus, phage or plasmid), then transplanting the cell into the body.

It has been demonstrated in cell and animal tests that *spink6* gene and the encoded protein described herein can significantly inhibit the proliferation of the tumor cells with lower toxicity, without affecting the normal functions of the animal tissues and organs, have good anti-liver cancer activity, and provide a safe and effective gene therapy drug for the clinical treatment of cancers.

## DESCRIPTION OF THE FIGURES

Figure 1 shows the level of *spink6* in the stable cell strain detected by RT-PCR, wherein the upper band is an amplification band of *spink6* and the lower band is an amplification band of GAPDH.

Lane 1: QGY-7703 cell
Lane 1: SP-vec cell
Lane 3: SP80 cell
Lane 4: SP57 cell

Figure 2 shows that *spink6* inhibits the growth of the QGY-7703 cell.

Figure 3 shows the tumor clone inhibition experiments on a soft agar.

Figure 4 shows the inhibition experiment in the nude mice bearing the tumor.

Figure 5 shows volumes of the tumors in the nude mice induced by four cell strains.

Figure 6 shows the prokaryotic expression and purification of the SPINK6 protein.

Lane M: the protein molecular weight standard
Lane 1: before induction
Lane 2: induction for 4 hours by IPTG
Lane 3: the recombinant protein after affinity purification using Ni Sepharose 6 FF (after desalination by dialysis, it was cleaved by enterokinase to obtain the SPINK6 protein).

Figure 7 shows the expression of SPINK6a protein in yeast and the purification.

Lane M: the protein molecular weight standard
Lane 1: the recombinant protein after purification
Lane 2: before induction
Lane 3~7: induction by methanol for 36, 48, 60, 72 and 96h, respectively.

Figure 8 shows the expression of SPINK6b protein in yeast and the purification.

Lane M: the protein molecular weight standard
Lane 1-2: the recombinant protein SPINK6b after purification

Figure 9 shows the determination of the Ad-SPINK6 virus titer via gradient infection.
1-5 are as follows respectively: 1, infection volume 1μl; 2, infection volume 2μl; 3, infection volume 5μl; 4, infection volume 10μl; 5, infection volume 15μl.

Figure 10 shows the growth curve of the tumor cells inhibited by the recombinant adenoviruses Ad-sp80 and Ad-sp57.

Figure 11 shows images of QGY-7703 after infection with the viruses Ad-sp80 and Ad-sp57 for 72h.

## MODES FOR CARRYING OUT THE INVENTION

The invention will now be further illustrated through the following Examples. It should be understood that these Examples are merely illustrative of the invention. The experimental methods without the specific experimental conditions in the following examples, are usually in accordance with the conventional conditions, such as those described in Sambrook et al., Molecular Cloning: A Laboratory Manual (New York: Cold Spring Harbor Laboratory Press, 1989), or in accordance with the recommended conditions by manufacturers.

### Example 1: The preparation of human *spink6* gene

In this example, human ***spink6*** cDNA fragment was amplified by RT-PCR method from the total RNA of the human liver cancer cell line QGY-7703.
The QGY-7703 cell was purchased from Cell Bank of Type Culture Collection Committee of Chinese Academy of Sciences/Center of Cell Resource, Shanghai Institute of life sciences of Chinese Academy of Sciences.

1. Extraction of total RNA

QGY-7703 cells in one 60 mm Petri dish were washed twice with PBS, after which 1 ml Trizol reagents for extracting RNA were added to sufficiently lyse the cells, and then were transferred into a 1.5 ml centrifugal tube. After adding 200 μl of chloroform and mixing homogeneously, centrifugation was performed at 12000 RPM for 10 min. The upper aqueous phase was transferred to another centrifugal tube, and then equal amount of isopropanol was added, allowing it standing at room temperature for 10 min and centrifugating at 12000 RPM for 10 min to obtain the total RNA pellets. The pellets were washed twice with 500 μl 75% ethanol, then dissolved in RNase-free de-ionized water. The quality identification of the extracted RNA was performed as follows: determining 260/280 ratio by UV spectrophotometer (ratio of 1.7 to 2.0); and observing the presence or absence of degradation in the MOPS formaldehyde denaturing gel.

2. Synthesis of cDNA

2μg of total RNA, 1 μl of oligo d(T) and 5 μl of $ddH_2O$ were incubated at 70 °C for 5 min, and then transferred immediately on ice for denaturation for 5 min. Then 4 μl of 5 x buffer, 2 μl of dNTPs (10 mM), 0.5 μl of RNasin, 1.5 μl of $ddH_2O$ and 1 μl of M-MLV (TaKaRa, D2640A) were added and mixed thoroughly, and incubated at 42 °C for 1 h, and finally, incubated at 70 °C for 15 min, and cooled on ice immediately to terminate the reaction.

3. PCR amplification

Primers were designed and synthesized based on the sequence of human *spink6* gene. In order to insert the PCR product directly into the eukaryotic expression vector pcDNA3.1(+) (Invitrogen, USA), wherein *EcoR I* and *Hind III* restriction site (in italics) were designedly added into 5' primer and 3' primer respectively, and the sequences of the primers are as follows:

The primers for the sequence of *spink6* gene encoding the full length of 80 amino acids are as follows:

Spink6(F): 5'CG***GAATTC***CGAATGAAACTGTCAGGCATG3'
Spink6(R): 5'CC***AAGCTT***TCAGCATTTTCCAGGATG3'

The primers for the sequence of *spink6* gene encoding the full length of 57 amino acids are as follows:

Spink6 (F): 5'CG ***GAATTC*** CAG GGA GGACAGGTTGAC 3'
Spink6(R): 5'CC ***AAGCTT*** TCAGCATTTTCCAGGATG3'

The PCR reaction mixture consisted of 10 x buffer, $MgCl_2$ (25 mM), dNTP (10 mM), spink6(F), spink6(R), Taq DNA polymerase, $ddH_2O$ and cDNA template with the volume of 2.5, 2.0, 0.5, 0.3, 0.3, 0.5, 17, 2μl, respectively. The reaction conditions were 5 min at 94 °C for denaturation, then 35 cycles of 30s at 94 °C, 30s at 55 °C and 30s at 72 °C for each cycle, and a final extension of 10 min at 72 °C. After the completion of the reaction, the PCR fragments obtained via verification contained the sequence of *spink6* gene encoding the full length of 80 amino acids and the sequence of *spink6* gene encoding 57 amino acids, respectively.

**Example 2: Screening and identification of QGY-7703 cell strain stably expressing *spink6***

1. Construction of plasmid

The PCR products obtained in Example 1 were respectively cloned into the vector pcDNA3.1(+) (Invitrogen, USA) with the drug G418 (an antibiotic, also known as geneticin, having a molecular weight 692.7Dalton, and routinely being used in screening for the selection of the resistant cells after transfection of neomycin resistant genes) resistant selection marker to construct expression plasmids pcDNA3.1(+)-sp80 and pcDNA3.1(+)-sp57, wherein the PCR products obtained in Example 1 were the sequence (SEQ ID NO:3) of *spink6* gene encoding the full length 80 of amino acids, and the gene sequence (SEQ ID NO:1) encoding 57 amino acids with the 23 amino acids signal peptide removed.

2. Cell transfection

(1) Before transfection, the cells were digested with 1% trypsin, and inoculated in the 24-well plate cultured in the medium without antibiotics for 24 h to ensure that cells were in the range of optimal transfection density (70 ~ 80%) (Lipofectamine 2000 kit, Invitrogen)
(2) 0.8 μg of the constructed plasmids pcDNA3.1(+)-sp80 and pcDNA3.1(+)-sp57 were mixed thoroughly with 50 μl of 1640 medium (free of serum and antibiotics, GIBCO), and the empty vector pcDNA3.1(+) was used as a negative control. Another 2 μl of Lipofectamine 2000 was thoroughly mixed with 50 μl of 1640 medium (free of serum and antibiotics, GIBCO) and then left standing for 5 min; finally the liposome diluent added to the plasmid diluent, mixing gently and kept at room temperature for 20 min.
(3) The cell culture supernatant was discarded, and the cells were washed once with PBS. After adding 400 μl of 1640 medium (free of serum-free and antibiotics), then the transfection mixture added dropwise after standing for 20 min, and shaken gently.

(4) 6 hrs after the transfection, the medium was replaced with fresh complete 1640 medium (containing serum and antibiotics, i.e., containing 10% fetal bovine serum and 100 U/ml penicillin, 100μg/ml streptomycin and 50μg/ml gentamicin) and the culture was continued to the total transfection time of 48 h.

3. Screening of stable strains

(1) After 48 h transfection, cells were digested and transferred to 100 mm Petri dishes and cultured for 24 h, G418 was further added to a final concentration of 600 μ/ml, and the culture was continued for 2 weeks until the formation of clones. Distinct single clones were picked with the cloning ring, transferred into the 96-well plates, and continued to culture with fresh complete 1640 culture medium (containing serum and antibiotics, i.e., containing 10% fetal bovine serum and 100 U/ml penicillin, 100μg/ml streptomycin and 50μg/ml gentamicin), where the G418 concentration was changed to 300 μg/ml, and then the culture was expanded continuously. A portion of cells were cryopreserved, and the rest continued to be expanded, after which the proteins and RNAs from cells were prepared for identification.

(3) The distinct single clones mentioned in step (1) were checked by RT-PCR, those strains expressing spink6 mRNA relatively higher than QGY-7703 cells, named SP80 cells and SP57 cells, were selected and could be used for checking the influence of cytological effects of *spink6* over-expression. At the same time, a cell strain transfected with the empty vector pcDNA3.1(+), designated as SP-vec cell, was screened out as well (Figure 1). It showed that the expression levels of *spink6* in the stable cell strains transfected with pcDNA3.1(+)-sp80 and pcDNA3.1(+)-sp57 were significantly higher than those of the control cells (the QGY-7703 cells and the cells transfected with the empty vector pcDNA3.1 (+)).

**Example 3: Effect of *spink6* expression on cell growth**

Four types of cells, SP80, SP57, SP-vec and QGY-7703 were inoculated into the 96-well plates in a density of 5 x $10^3$ cells/well. The cell growth state was measured every 24h with a kit (Promega, CellTiter 96 AQueous Non-Radioactive Cell Proliferation Assay) and the cell growth curve was shown in Figure 2. Figure 2 showed that the growth rate of the stable cell strain highly expressing spink6 was significantly slower than that of the control cells (QGY-7703 cells and the cells transfected with the empty vector pcDNA3.1(+)), indicating that high expression of SPINK6 protein with or without the signal peptide of 23 amino acids could significantly inhibit the growth of QGY-7703 cells.

**Example 4: Detection of effect of *spink6* on cell cycle by flow cytometry**

The SP80, SP57, SP-vec and QGY-7703 cells in Example 2 were inoculated into the 6-well plates in a density of 1 x $10^5$ cells/well. 24h after inoculation, the medium was replaced with the 1640 medium (free of serum and antibiotics) and cultured for 24 h to allow cell synchronization, then in complete 1640 medium (containing serum and antibiotics) for 24 h, after which the cells were collected and fixed in 75% ethanol for more than 24 h. The fixed cells were washed twice with PBS, stained with PI (propidium iodide, a fluorescent dye for staining the nucleus, and emitting red fluorescence after being embedded into the double-stranded DNA) for 30 min. Finally the distribution profile of the four kinds of cells in various cell division phases was checked by flow cytometry (FACS) (Table 1). In table 1, G1 phrase is the DNA pre-synthetic phase, the early stage of G1 phase is G0 phase, G0-G1 represents the DNA pre-synthetic phase; S phrase is the DNA synthetic phase; G2 phrase is the DNA post-synthetic phase, M phase is the mitotic phase, and G2-M represents after the synthesis of DNA until the next round of DNA pre-synthetic phase. The results indicated that, in contrast of QGY-7703 cells, the ratio of the SP80 and SP57 cells highly expressing the two SPINK6 proteins in different lengths at post-synthetic phase was increased by two folds, which shows that the two SPINK6 proteins of different lengths are both capable of resulting in the QGY-7703 cells to be arrested in G2/M phase and lowering the cell proliferation ability.

**Table 1 Cell cycle profiles checked by flow cytometry**

| Cell group / cycle | QGY-7703 | pcDNA3.1(+) | SP80 | SP57 |
|---|---|---|---|---|
| G0-G1 | 68.01 | 68.74 | 64.15 | 63.59 |
| G2-M | 9.44 | 9.39 | 17.38 | 16.94 |
| S | 22.55 | 21.87 | 18.46 | 19.46 |

**Example 5: The inhibition test of tumor cell clone formation on soft agar**

3 ml per dish of 1640 medium containing 10% fetal bovine serum, 0.6% agar by weight, 100 U/ml penicillin, 100μg/ml streptomycin and 50μg/ml gentamicin was prepared and added to 60 mm-diameter Petri dishes, solidified at room temperature. Then the single cell suspensions of the experimental group and the control group cells were prepared respectively and adjusted to the density of 1 x $10^5$ cells/ml, 40 μl of which was taken and mixed rapidly with 2 ml of 0.6% agar and 2 ml of 2 x 1640 complete medium (the concentrations of 1640, bovine serum and antibiotics are twice the concentrations of the normal 1640 medium) pre-incubated at 42 °C. 3 ml was plated on the bottom agar layer in triplicate for each sample. After solidified and cultured for 3 weeks in $CO_2$ incubator, the clone formation could be observed. The results were shown in Figure 3. It showed that the stable cell strains (SP80 and SP57) transfected with spink6 could not form clones on soft agar, while the control cells (SP-vec and QGY-7703) could form clones on soft agar efficiently, which indicates that the two SPINK6 proteins completely inhibit the growth of the transfected cells on soft agar.

**Example 6: Tumor inhibition experiments in nude mice**

Four cell strains, SP80, SP57, SP-vec and QGY-7703 in Example 2, were inoculated subcutaneously into 5-week-old nude mice with the amount of 2 x $10^5$ cells/animal. The tumor growth was examined every four days, and the results were shown in Figures 4 and 5. Figure 4 showed that the stable cell strains SP80 and SP57 could no longer induce the formation of tumor in nude mice, wherein the arrows indicate the sites of tumor formation. Figure 5 showed that SP80 and SP57 were not tumorigenic yet, and no tumors were formed in the inoculated nude mice, whereas tumors formed gradually over time after the nude mice were inoculated with the control (the QGY-7703 cells and the cells transfected with empty vector pcDNA3.1(+)). The results showed that the growth of the liver cancer cell QGY-7703 and the formation of tumors *in vivo* were inhibited significantly by the two SPINK6 proteins.

**Example 7: Induction, expression and purification of SPINK6 protein in *E. coli***

The synthetic gene fragment sp6 was digested with NcoI and HindIII restriction enzymes and inserted into the expression vector pET32a to construct the recombinant plasmid pET32aSP6.

The *spink6* gene fragment sp6 had the nucleotide sequence designated as SEQ ID NO:5:

gccatggcta aactgtcagg catgtttctg ctcctctctc tggctctttt ctgcttttta acaggtgtct

tcagtcaggg aggacaggtt gactgtggtg agttccagga caccaaggtc tactgcactc gggaatctaa

cccacactgt ggctctgatg gccagacata tggcaataaa tgtgccttct gtaaggccat agtgaaaagt

ggtggaaaga ttagcctaaa gcatcctgga aaatgctgaa gctt

After the transformation of BL21 (DE3) by the recombinant plasmid, the transformants were inoculated into 2mL LB medium and cultured overnight at 37 °C. 1 mL of the broth was inoculated into 100mL LB medium, and cultured at 37 °C until OD600=0.8, at which IPTG was added to a final concentration of 1mmol/L. After 4h induction, the expression profile was checked by SDS-PAGE electrophoresis (Figure 6). It showed the expression of the recombinant SPINK6 in BL21 (DE3):

Lane M: the protein molecular weight standard
Lane 1: before induction
Lane 2: IPTG induction for 4 hours
Lane 3: the recombinant protein after affinity purification using Ni Sepharose 6 FF. After desalination by dialysis, it was cleaved by enterokinase to obtain the SPINK6 protein.

**Example 8: SPINK6 protein induction, expression and purification in yeast**

The synthetic gene fragments spink6a and spink6b containing spink6 gene were digested with *XhoI* and *EcoRI* restriction enzymes and inserted into the plasmid pPIC9 to obtain the recombinant plasmids pPIC9-SP6a and pPIC9-SP6b.

The gene fragment spink6a had the nucleotide sequence designated as SEQ ID NO:6:

ctcgagaaaa gacagggtgg acaggttgac tgtggtgagt tccaggaccc aaaggtctac tgcactagag agtctaaccc

acactgtggt tctgatggac agacttacgg caacaaatgt gccttctgta aggccatagt taagtctggt ggaaagattt

ctctgaagca tcctggaaag tgctaatgag aattc

The gene fragment spink6b had the nucleotide sequence designated as SEQ ID NO:7:

ctcgagaaaa gaatgaaact gtcaggcatg tttctgctcc tctctctggc tcttttctgc tttttaacag gtgtcttcag

tcagggtgga caggttgact gtggtgagtt ccaggaccca aaggtctact gcactagaga gtctaaccca cactgtggtt

ctgatggaca gacttacggc aacaaatgtg ccttctgtaa ggccatagtt aagtctggtg gaaagatttc tctgaagcat

cctggaaagt gctaatgaga attc

pPIC9-SP6a and pPIC9-SP6b were digested with *Bam*HI and *Eco*RI, and the released fragments were inserted into the expression vector pPIC9K to obtain the recombinant plasmids pPIC9K-SP6a and pPIC9K-SP6b, respectively. The derived plasmids were electroporated into *P. pastoris* GS115. The transformed cells were selected on MD plates. Subsequently, the high-copy transformants were screened out by YPD-G418.

The selected high-copy transformants of pPIC9K-SP6a and pPIC9K-SP6b were inoculated into 25ml BMGY medium and cultured overnight at 30 °C. The cells were collected by centrifugation x3000g. The pellet was resuspended in the BMMY medium to reach $OD_{600}$ around 1.0, then cultured at 28 ~ 30 °C. Methanol was added every 24 h to a final concentration of 0.5% (v/v) to induce expression of the target protein. After induction, for 72h, the expression profiles for SPINK6a (Figure 6) and SPINK6b (Figure 7) were checked by SDS-PAGE.

Figure 6 showed the expression of the recombinant SPINK6a in GS115:

Lane M: the standard protein molecular weight
Lane 1: the purified recombinant protein SPINK6a
Lane 2: before induction
Lane 3~7: methanol induction for 36, 48, 60, 72 and 96h, respectively.

The purified protein was structurally confirmed as the SPINK6 protein designated as SEQ ID NO:2.

Figure 7 showed the expression of the recombinant SPINK6b in GS115:

Lane M: the protein molecular weight standard
Lane 1~2: the purified recombinant protein SPINK6b

The purified protein was structurally confirmed as the SPINK6 protein designated as SEQ ID NO:4.

**Example 9: Preparation of SPINK6 Protein Injection**

5ml of sterile water were added to 1 mg of the soluble recombinant SPINK6a protein and 1 mg of the soluble recombinant SPINK6b protein obtained in Example 8 to prepare the injection in a dosage of 200 μg/ml.

**Example 10: Activity of inhibition proliferation of the tumor cells *in vivo* by the soluble recombinant SPINK 6 protein**

2 x $10^6$ QGY-7703 cells were inoculated subcutaneously into the nude mice. The diameters of the transplanted tumors were measured with vernier caliper 2 to 3 times every week. When the tumor was grown to $100mm^3$, the tumor animal model of the nude mice bearing QGY-7703 liver cancer cells was prepared successfully. The injections of the soluble recombinant SPINK6a and SPINK6b proteins prepared in Example 9 were administered intratumorally in a dosage of 0.1, 0.5, 1.0 mg/kg/day respectively, with the physiological saline as a negative control. Injection was performed every other day for 14 days. The mice were sacrificed at the time of 7 days after stopping the administration, and the weight of the tumor mass was measured.

The result (Table 2) shows that *spink6* can significantly inhibit the proliferation of QGY-7703 liver cancer cells in the nude mice.

**Table 2 Tumor inhibition of the nude mice by soluble recombinant SPINK6 protein (n = 5)**

| Group | Dosage (mg/kg) | Weight of tumor (g) | Inhibition rate of tumor (%) |
|---|---|---|---|
| Control | -- | 1.624±0.31 | -- |
| rhs SPINK6a protein | 0.1<br>0.5<br>1.0 | 1.573±0.40<br>1.121±0.26<br>0.854±0.39 | --<br>31.0<br>47.4 |
| rhs SPINK6b protein | 0.1<br>0.5<br>1.0 | 1.482±0.35<br>1.050±0.29<br>0.779±0.27 | --<br>35.3<br>52.0 |

**Example 11: Construction of recombinant adenovirus vector (Ad-SPINK6) carrying the SPINK6**

The sequence of human spink6 gene (SEQ ID NO:3) encoding the whole length of 80 amino acids and the sequence of the gene (SEQ ID NO:1) encoding the 57 amino acids with the signal peptide of 23 amino acids removed were amplified via PCR, and the conditions for PCR are the same as "3. PCR amplification" in Example 1.The primers for PCR are as follows:

The primers for the amplification of SEQ ID NO:3 are as follows:

Forward: 5'CC GGTACC ATG AAA CTG TCA GGC ATG 3'
Reverse: 5'CG CTCGAG TCA GCA TTT TCC AGG ATG 3'

The primers for the amplification of SEQ ID NO:1 are as follows:

Forward: 5'CC GGTACC CAG GGA GGA CAG GTT GAC 3'
Reverse: 5'CG CTCGAG TCA GCA TTT TCC AGG ATG 3"

The amplified fragment via PCR was double-digested using XhoI and KpnI restriction enzymes and ligated into pAdTrack to obtain the recombinant plasmids pAdTrack-sp80 and pAdTrack-sp57, and then were linearized by PmeI and transformed into *E.coli* BJ5183 containing pAdEasy plasmid for recombination. The positive transformants were identified by *PacI* restriction enzyme single digestion, and the larger fragment recovered. The larger fragment was encapsulated in liposome, transfected into 293T cells for virus packaging, expression and amplification, with an empty virus Ad-GFP being amplified synchronously as control. Finally, a sufficient amount of recombinant viruses Ad-sp80 and Ad-sp57 and the empty virus Ad-GFP were recovered, frozen and thawed and then subpackaged, to ensure the uniform concentration and titer in each tube. The specific methods of operation can be found in the reference (A protocol for rapid generation of recombinant adenoviruses using the ADEasy system [J]. Nature Protocols, 2007, 2:1236-1247).

The gradient transfection was carried out with the recombinant adenovirus and the empty virus in the 24-well plates. After 24 h, the average virus infection volume (μL/well) with 100% fluorescence was taken, and the number of 293T cells in the wells was counted, and the viral titer was calculated using follow formula and was expressed as PFU (plaque formation unit, representing the number of infectious or living viruses).

$$\text{Viral titer} = \text{Number of cells}/\text{Average virus infection volume}$$

The results were shown in Figure 9. It showed that 24 hours after the infection of 293T cells with the recombinant adenovirus Ad-SPINK6 in different volumes, fluorescent spots as the light color spots were detected in the cells. The results showed that the higher the volume of the recombinant adenovirus was, the more cells with fluorescent spots were. When the volume of infection was between 10μl and 15μl, all the 293T cells were infected.

**Example 12: Activity of inhibition proliferation of tumor cells by recombinant adenovirus Ad-sp80 and Ad-sp57**

The QGY-7703 cells were inoculated into the 96-well plates in a density of 5 x $10^3$cells/well. A multiplicity of infection 10 (MOI 10) of the recombinant adenoviruses Ad-GFP (empty virus), Ad-sp80 and Ad-sp57 obtained in Example 11 was selected for the infection of QGY-7703 cells inoculated in the 96-well plates. The cell growth state was detected every 24 h with the kit (Promega, CellTiter 96 AQueous Non-Radioactive Cell Proliferation Assay), and the cell growth curve was depicted as shown in Figure 10. It showed that the growth rates of QGY-7703 cells infected with Ad-sp80 and Ad-sp57 were significantly lower than those of the control groups (PBS group and Ad-GFP group), indicating that Ad-sp80 and Ad-sp57 could significantly inhibit the growth of QGY-7703 cells. The images of the infected cells were shown in Figure 11. It showed that after infected by the empty virus Ad-GFP, the growth of QGY-7703 was substantially not affected, while after infected by Ad-sp80 and Ad-sp57, the growth rate of QGY-7703 was significantly reduced.

**Example 13: Acute toxicity test of SPINK6 protein in mice**

Twenty of KM mice, half male and half female, were singly injected the recombinant soluble SPINK6a and SPINK6b proteins obtained in Example 8 in the maximum concentration (10%) and the maximum dosing volume (25 ml/kg) via the tail vein. The administration was carried out once, and the observation was carried out continuously for 2 weeks. After the administration, observation was carried out continuously for 30 min, and once an hour for 4 hours, then once every morning and evening. The results indicated that the mice did not show discernable symptoms of poisoning after the injection of the recombinant SPINK6a and SPINK6b proteins via the tail vein, and none of the 20 animals died.

## Claims

1. Use of the *spink6* gene or its encoded protein in the manufacture of an anti-liver cancer drug, wherein the gene has a nucleotide sequence designated as SED ID NO:1, and the encoded protein by the gene has an amino acid sequence designated as SED ID NO:2.

2. The use according to claim 1, which is **characterized in that** the gene further comprises a sequence of 69 nucleotides encoding a signal peptide of 23 amino acids at 5' end of the nucleotide sequence designated as SED ID NO:1, and has a nucleotide sequence designated as SED ID NO:3, and that the protein encoded by the gene further comprises a signal peptide of 23 amino acids at N-terminus of the amino acid sequence designated as SED ID NO:2, and has an amino acid sequence designated as SED ID NO:4.

3. The use according to claim 1, which is **characterized in that** the drug is a pharmaceutical composition comprising the amino acid sequence designated as SED ID NO:2.

4. The use according to claim 1, which is **characterized in that** the drug is a pharmaceutical composition of the recombinant gene therapy vector comprising the nucleotide sequence designated as SED ID NO:1.

5. The use according to claim 2, which is **characterized in that** the drug is a pharmaceutical composition comprising the amino acid sequence designated as SED ID NO:4.

6. The use according to claim 2, which is **characterized in that** the drug is a pharmaceutical composition of the recombinant gene therapy vector comprising the nucleotide sequence designated as SED ID NO: 3.

## Patentansprüche

1. Verwendung des *spink6*-Gens oder seines codierten Proteins bei der Herstellung eines Medikaments gegen Leberkrebs, wobei das Gen eine als SED ID NO:1 bezeichnete Nukleotidsequenz hat und das durch das Gen codierte Protein eine als SED ID NO:2 bezeichnete Aminosäuresequenz hat.

2. Verwendung nach Anspruch 1, die **dadurch gekennzeichnet ist, dass** das Gen ferner eine Sequenz von 69 Nukleotiden umfasst, die ein Signalpeptid aus 23 Aminosäuren am 5'-Ende der als SED ID NO:1 bezeichneten Nukleotidsequenz codiert, und eine als SED ID NO:3 bezeichnete Nukleotidsequenz hat, und dass das durch das Gen codierte Protein ferner ein Signalpeptid aus 23 Aminosäuren am N-Terminus der als SED ID NO:2 bezeichneten Aminosäuresequenz umfasst und eine als SED ID NO:4 bezeichnete Aminosäuresequenz hat.

3. Verwendung nach Anspruch 1, die **dadurch gekennzeichnet, ist, dass** das Medikament eine pharmazeutische Zusammensetzung ist, welche die als SED ID NO:2 bezeichnete Aminosäuresequenz umfasst.

4. Verwendung nach Anspruch 1, die **dadurch gekennzeichnet, ist, dass** das Medikament eine pharmazeutische Zusammensetzung des rekombinanten Gentherapievektors ist, der die als SED ID NO:1 bezeichnete Nukleotidsequenz umfasst.

5. Verwendung nach Anspruch 2, die **dadurch gekennzeichnet ist, dass** das Medikament eine pharmazeutische Zusammensetzung ist, welche die als SED ID NO:4 bezeichnete Aminosäuresequenz umfasst.

6. Verwendung nach Anspruch 2, die **dadurch gekennzeichnet ist, dass** das Medikament eine pharmazeutische Zusammensetzung des rekombinanten Gentherapievektors ist, der die als SED ID NO:3 bezeichnete Nukleotidsequenz umfasst.

**Revendications**

1. Utilisation du gène *spink6* ou de sa protéine codée dans la fabrication d'un médicament anti-cancer du foie, dans laquelle le gène a une séquence nucléotidique désignée par SEQ ID NO : 1, et la protéine codée par le gène a une séquence d'acides aminés désignée par SEQ ID NO : 2.

2. Utilisation selon la revendication 1, qui est **caractérisée en ce que** le gène comprend en outre une séquence de 69 nucléotides codant pour un peptide signal de 23 acides aminés à l'extrémité 5' de la séquence nucléotidique désignée par SEQ ID NO : 1, et a une séquence nucléotidique désignée par SEQ ID NO : 3, et **en ce que** la protéine codée par le gène comprend en outre un peptide signal de 23 acides aminés à l'extrémité N-terminale de la séquence d'acides aminés désignée par SEQ ID NO : 2, et a une séquence d'acides aminés désignée par SEQ ID NO : 4.

3. Utilisation selon la revendication 1, qui est **caractérisée en ce que** le médicament est une composition pharmaceutique comprenant la séquence d'acides aminés désignée par SEQ ID NO : 2.

4. Utilisation selon la revendication 1, qui est **caractérisée en ce que** le médicament est une composition pharmaceutique du vecteur recombinant de thérapie génique comprenant la séquence nucléotidique désignée par SEQ ID NO : 1.

5. Utilisation selon la revendication 2, qui est **caractérisée en ce que** le médicament est une composition pharmaceutique comprenant la séquence d'acides aminés désignée par SEQ ID NO : 4.

6. Utilisation selon la revendication 2, qui est **caractérisée en ce que** le médicament est une composition pharmaceutique du vecteur recombinant de thérapie génique comprenant la séquence nucléotidique désignée par SEQ ID NO : 3.

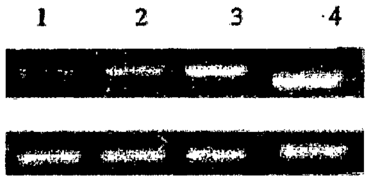


**Figure 1**

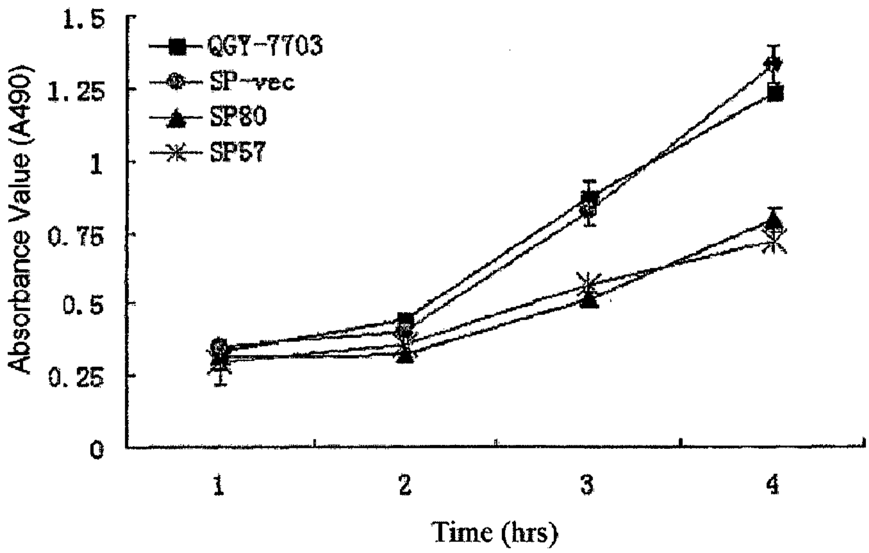


**Figure 2**

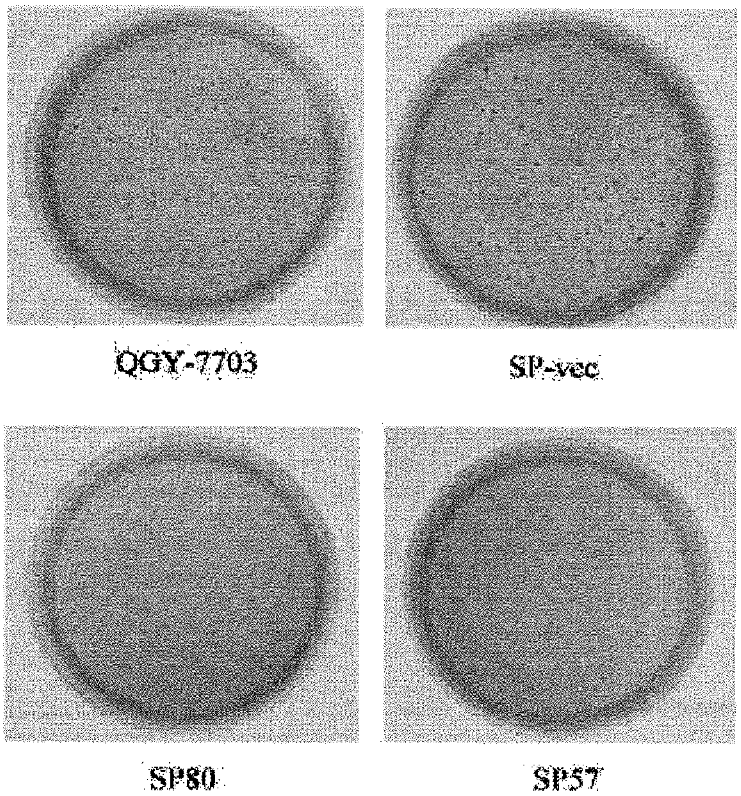


**Figure 3**

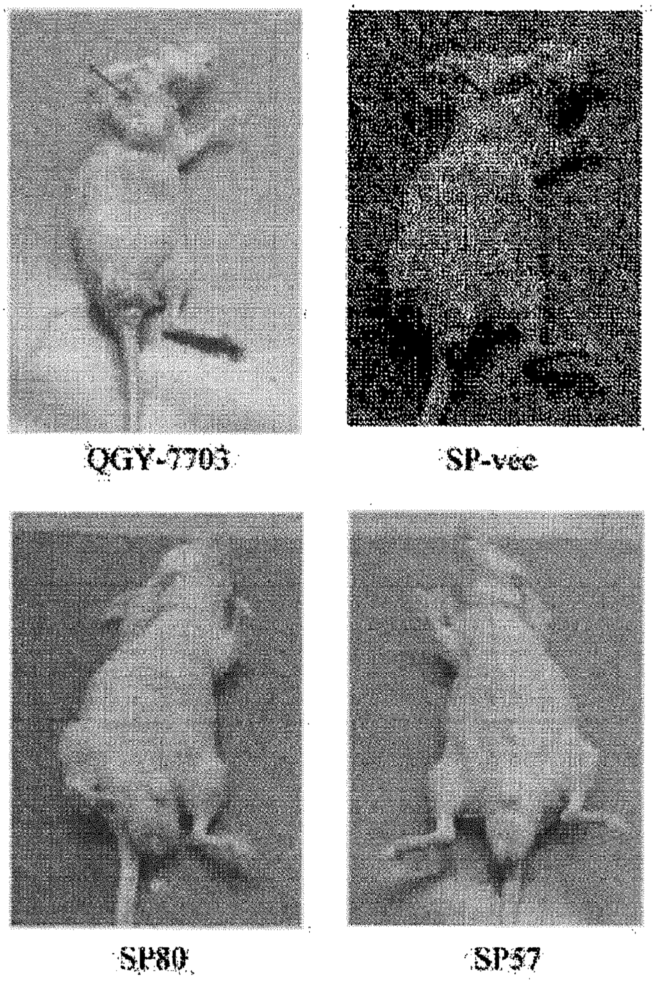


**Figure 4**

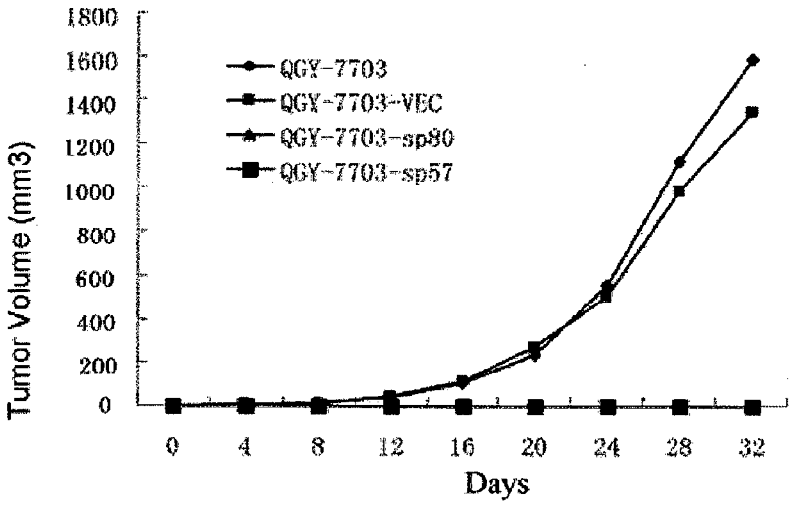


**Figure 5**

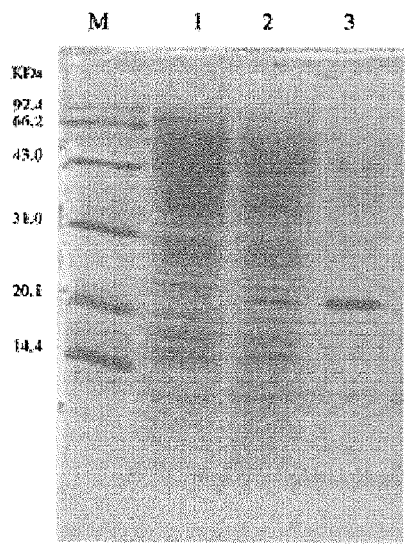


**Figure 6**

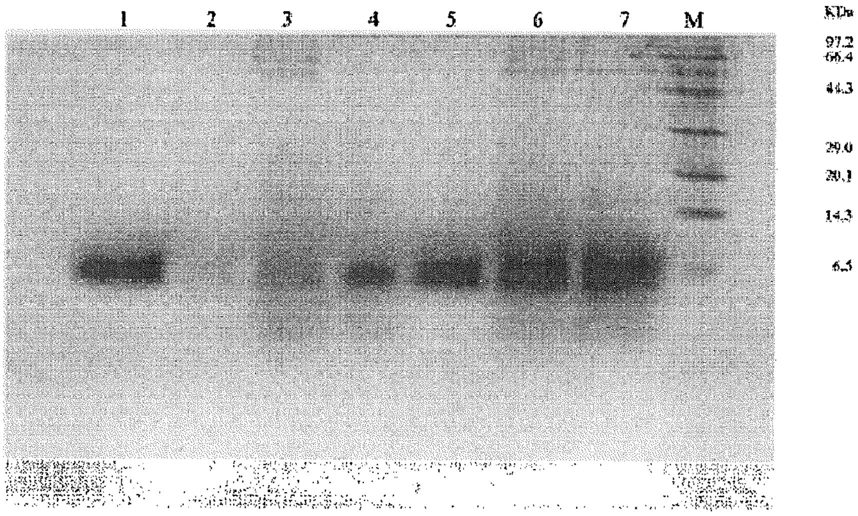


**Figure 7**

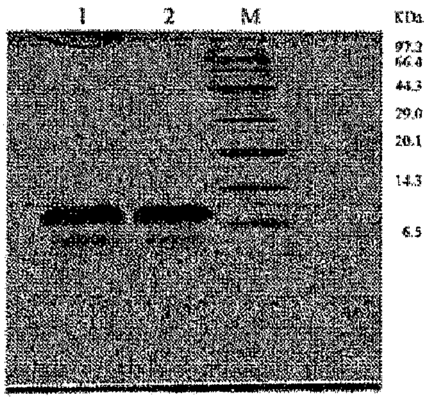


**Figure 8**

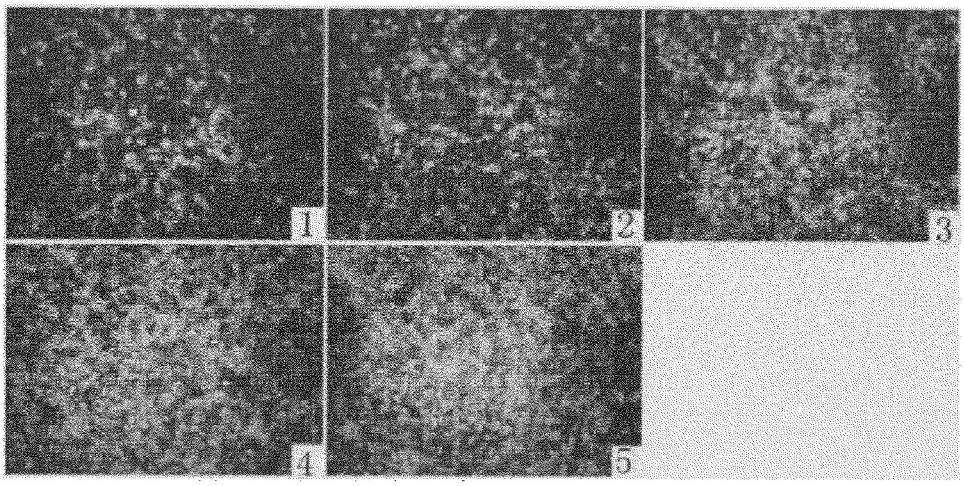


**Figure 9**

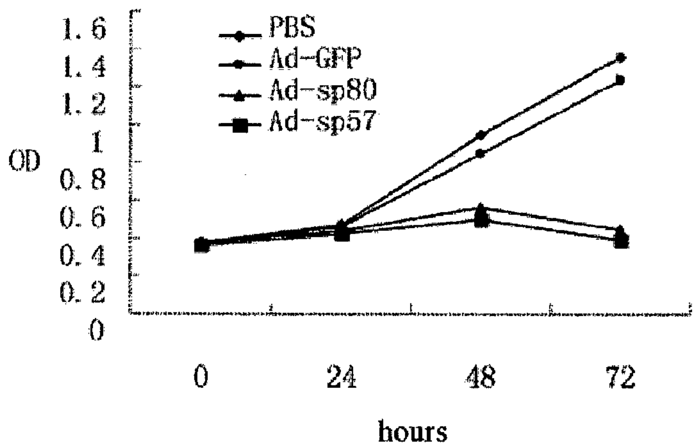


**Figure 10**

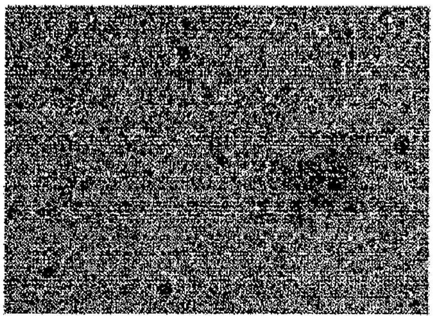

PBS-treatment group,

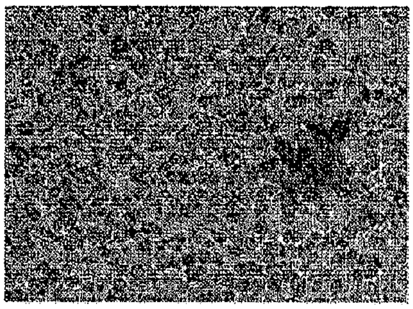

Ad-GFP, X200

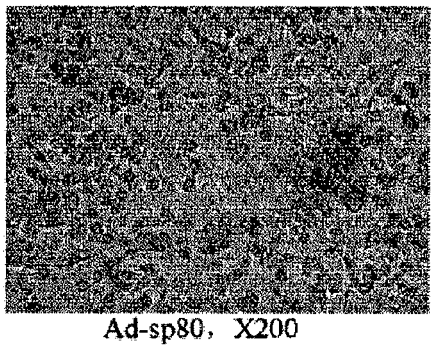

Ad-sp80, X200

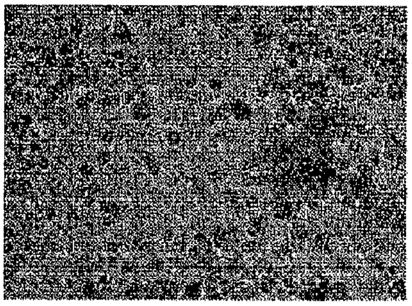

Ad-sp57, X200

**Figure 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2009074410 W


### Non-patent literature cited in the description


- **MARTIN C. WAPENAAR ; ALIENKE J. MONSUUR et al.** *Immunogenetics,* 2007, vol. 59, 349-357
- **MICHAEL D. OBERST ; MICHAEL D. et al.** *Clinical Cancer Research,* April 2002, vol. 8, 1101-1107
- **KLEZOVITCH J. ; CHEVILLET J. et al.** *Cancer Cell,* 2004, vol. 6 (2), 185-195
- *Science,* 1984, vol. 224, 1431
- **JOE SAMBROOK.** Molecular Cloning: A Laboratory Manual
- **SAMBROOK et al.** Molecular Cloning: A Laboratory Manual. Cold Spring Harbor Laboratory Press, 1989
- A protocol for rapid generation of recombinant adenoviruses using the ADEasy system [J. *Nature Protocols,* 2007, vol. 2, 1236-1247